(19) Europäisches Patentamt
European Patent Office
Office européen des brevets

(11) Numéro de publication: **0 171 320**
**B1**

(12) **FASCICULE DE BREVET EUROPEEN**

(45) Date de publication du fascicule du brevet: **28.10.87**

(51) Int. Cl.[4]: **C 07 C 67/317**, C 07 C 69/738

(21) Numéro de dépôt: **85401412.3**

(22) Date de dépôt: **11.07.85**

(54) **Procédé de préparation de composés insaturés chlorés en alpha de deux groupements électroattracteurs en position bêta.**

(30) Priorité: **12.07.84 FR 8411088**

(43) Date de publication de la demande: **12.02.86 Bulletin 86/7**

(45) Mention de la délivrance du brevet: **28.10.87 Bulletin 87/44**

(84) Etats contractants désignés: **AT BE CH DE FR GB IT LI LU NL SE**

(56) Documents cités: **EP - A - 0 082 781**

(73) Titulaire: **RHONE-POULENC SANTE, Les Miroirs 18 Avenue d'Alsace, F-92400 Courbevoie Cédex (FR)**

(72) Inventeur: **Mignani, Gérard, 2 avenue des Frères Lumière, F-69008 Lyon (FR)**
Inventeur: **Morel Didier, 3 rue Jean Jaurès, F-91700 Villiers-sur-Orge (FR)**

(74) Mandataire: **Pilard, Jacques et al, RHONE-POULENC RECHERCHES Service Brevets Pharma 25, Quai Paul Doumer, F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 171 320 B1

**Description**

La présente invention concerne un procédé de préparation de composés insaturés chlorés en α de deux groupements électroattracteurs en position β de formule générale (Ia) ou (Ib) dans laquelle n représente un nombre entier entre 1 et 10 inclusivement et X et Y, identiques ou différents, représentent chacun un groupement électroattracteur choisi parmi les radicaux -CHO, $-COR_1$, $-COOR_2$, $-CONR_3R_4$, -CN, $-SO_2R_5$ et $NO_2$.

Dans ce qui précède et ce qui suit, les radicaux $R_1$, $R_2$, $R_3$, $R_4$ et $R_5$ représentent des radicaux alcoyles contenant 1 à 4 atomes de carbone, $R_5$ pouvant en outre représenter un radical phényle éventuellement substitué par un radical méthyle.

D'un intérêt tout particulier sont les produits de formule générale (I) dans laquelle l'un des symboles X ou Y représente un radical $-COR_1$ et l'autre un radical $-COOR_2$.

Dans la demande de brevet européen EP 0 082 781 a été décrit un procédé de préparation d'α-chloro-β-cétoesters par action du chlorure cuivrique, en présence de chlorure de lithium, dans un solvant aprotique polaire basique, tel que la N-méthylpyrrolidone, à une température comprise entre 15 et 50°C sur le β-cétoester correspondant.

D'après P.L. Stotter et K.A. Hill, Tetrahedron Letters, n° 40 4067 (1972), il est connu de préparer des α-bromo-β-cétoesters à partir des β-cétoesters correspondants par action de l'hydrure de sodium suivie de l'action rapide du brome dans le chlorure de méthylène à 0°C.

Cependant, il est connu, en particulier d'après M.L. Poutsma, J. Amer. Chem. Soc., 87 (19) 4285 (1965), que la chloration de composés éthyléniques par le chlore en présence d'oxygène à température ambiante conduit essentiellement à des produits d'addition accompagnés de produits de substitution allylique.

Selon le brevet américain US 2 995 600, la chloration du mycrène par le chlore gazeux conduit à la formation de chlor-3 méthyl-2 méthylène-6 octadiène-1,7.

Il est connu également, d'après le brevet américain US 4 272 412, que la chloration de la dihydro α-ionone par un acide hypohalogéneux préparé in situ conduit à la (chloro-5 méthylène-6 diméthyl-2,2 cyclohexyl)-4 butanone-2 sans formation de produit chloré en α de la fonction cétone.

Par ailleurs, lorsque l'on fait réagir un agent d'halogénation tel que le chlore moléculaire ou le chlorure de sulfuryle dans un solvant aprotique polaire sur un composé de formule générale (IIa) ou (IIb) dans laquelle n, X et Y sont définis comme précédemment, il se forme uniquement des produits d'halogénation de la chaîne terpénique sans observer la formation de composé chloré en α des groupements X et Y.

Il a maintenant été trouvé, et c'est ce qui fait l'objet de la présente invention, que les produits de formule générale (Ia) ou (Ib) peuvent être obtenus par action d'un agent d'halogénation sur un produit de formule générale (IIa) ou (IIb) anionisé au moyen d'une base.

Pour la mise en œuvre du procédé selon l'invention, il est particulièrement avantageux d'ajouter au produit de formule générale (IIa) ou (IIb) en solution dans un solvant organique anhydre convenable l'agent d'anionisation et l'agent d'halogénation.

Il est aussi possible d'effectuer l'anionisation du produit de formule générale (IIa) ou (IIb) puis d'ajouter ultérieurement l'agent d'halogénation.

Comme agents d'anionisation qui conviennent particulièrement bien peuvent être cités les hydrures, amidures, hydroxydes et carbonates de métal alcalin ou alcalino-terreux (sodium, potassium, calcium), les amines non quaternisables (1,8-diazabicyclo [5,4,0] undec-7-ène (DBU), collidines, éthyldiisopropylamine, éthyldicyclohexylamine).

Les solvants organiques peuvent être choisis parmi les hydrocarbures alphatiques (pentane, hexane), les hydrocarbures aromatiques (benzène, toluène), les éthers (éther éthylique, méthyltertiobutyléther, tétrahydrofuranne), les cétones (acétone, méthylisobutylcétone), les amides tertiaires (diméthylformamide, N-méthylpyrrolidone), les nitriles (acétonitrile), les solvants nitrés (nitrobenzène, nitrométhane) ou, lorsque l'on opère en présence d'un catalyseur de transfert de phase tel que l'hydrogénosulfate de tétrabutylammonium, les solvants polychlorés (chlorure de méthylène).

Les agents d'halogénation sont choisis parmi les sources de cations $Cl^+$ et plus particulièrement parmi le chlore moléculaire, le chlorure de sulfuryle, le N-chlorosuccinimide et l'hexachloroéthane.

Généralement, le procédé est mis en œuvre à une température comprise entre 0 et 120°C et, de préférence, voisine de 25°C.

Le procédé selon l'invention peut aussi être mis en œuvre en utilisant comme agent d'anionisation un alcoolate de métal alcalin (méthylate de sodium) dans l'alcool correspondant (méthanol). Cependant, dans ces conditions, l'anionisation et l'halogénation doivent être effectuées, de préférence, à une température inférieure à −30°C et plus particulièrement à une température voisine de −50°C.

Généralement on utilise une quantité stoechiométrique de l'agent d'anionisation et de l'agent d'halogénation par rapport au produit de formule générale (IIa) ou (IIb) mis en œuvre.

Les produits de formule générale (Ia) ou (Ib) obtenus selon le procédé de la présente invention peuvent être isolés du mélange réactionnel selon les méthodes habituelles d'extraction et ils peuvent être purifiés par application des méthodes physicochimiques telles que la distillation ou la chromatographie.

Les produits de formule générale (IIa) ou (IIb) peuvent être obtenus par addition sélective d'un composé à groupement méthylène actif de formule générale (III) dans laquelle X et Y sont définis comme précédemment, sur un butadiène de formule générale (IV) dans laquelle n est défini comme précédemment, dans les conditions décrites dans le brevet européen EP 0 044 771.

Les produits de formule générale (Ia) ou (Ib) sont particulièrement intéressants comme intermédiaires en chimie organique. Par exemple, les produits de formule générale (Ia) ou (Ib) dans laquelle l'un des symboles X ou Y représente un radical $-COCH_3$ et l'autre un radical $-COOR_2$ peuvent conduire facilement à des cétones éthyléniques de formule générale

(V) dans laquelle n est défini comme précédemment, sous forme d'un mélange des isomères EE ou EZ.

Le procédé de préparation des produits de formule générale (V) consiste à décarboxyler le produit de formule générale (Ia) ou (Ib) dans laquelle un des symboles X ou Y représente un radical $-COCH_3$ et l'autre un radical $-COOR_2$ pour obtenir une α-halogénocétone de formule générale (VIa) ou (VIb) dans laquelle n est défini comme précédemment qui est déhydrohalogénée pour obtenir le produit de formule générale (V).

Les réactions de décarboxylation et de déhydrohalogénation peuvent être effectuées sans isolement de l'α-halogénocétone de formule générale (VIa) ou (VIb).

Les réactions de décarboxylation et de déhydrohalogénation, successives ou simultanées, peuvent être réalisées dans les conditions décrites dans la demande de brevet européen EP 0 082 781.

Par exemple, le produit de formule générale (V) dans laquelle n = 1 est la pseudo-ionone que peut être transformée en vitamine A selon les méthodes connues.

Les exemples suivants, donnés à titre non limitatif, montrent comment l'invention peut être mise en pratique.

*Exemple 1*

Dans un ballon de 100 cm3, on introduit, sous atmosphère d'argon, 20 cm3 de diméthylformamide anhydre et 2,52 g (0,01 mole) d'un mélange 55/45 de:

$(CH_3)_2C=CH-CH_2CH_2-C(=CH_2)-CH_2CH_2-CH(COOCH_3)COCH_3$
et
$(CH_3)_2C=CH-CH_2CH_2-C(CH_3)=CH-CH_2-CH(COOCH_3)COCH_3$

puis on ajoute 0,6 g (0,0107 mole) de potasse broyée et 2,5 g (0,0105 mole) d'hexachloroéthane. On agite pendant 12 heures à 25°C. Le mélange réactionnel est versé dans 100 cm3 d'eau puis on extrait par 3 fois 30 cm3 d'éther éthylique. Les phases organiques sont réunies et séchées sur sulfate de magnésium. Après filtration et évaporation du solvant, on obtient 3,75 g d'une huile légèrement jaune. Par distillation sous pression réduite (0,1 mm de mercure; 0,012 kPa) à 150°C, on obtient 2,5 g d'un mélange 55/45 de:

$(CH_3)_2C=CH-CH_2CH_2-C(=CH_3)CH_2CH_2-CCl(COOCH_3)COCH_3$
et
$(CH_3)_2C=CH-CH_2CH_2-C(CH_3)=CH-CH_2-CCl(COOCH_3)COCH_3$

dont le titre est de 81%.

Le rendement en produits isolés est de 70%.

*Exemple 2*

Dans un ballon de 100 cm3, on introduit, sous atmosphère d'argon, 50 cm3 de N-méthylpyrrolidone et 5,5 g (0,0218 mole) d'un mélange 55/45 de:

$(CH_3)_2C=CH-CH_2CH_2-C(=CH_2)-CH_2CH_2-CH(COOCH_3)COCH_3$
et
$(CH_3)_2C=CH-CH_2CH_2-C(CH_3)=CH-CH_2-CH(COOCH_3)COCH_3$

puis on ajoute 2,49 g (0,0235 mole) de carbonate de sodium et 5,24 g (0,0221 mole) d'hexachloroéthane. On agite pendant 22 heures à 25°C. On observe la formation d'un précipité blanc. Après filtration, le mélange réactionnel est versé dans 150 cm3 d'eau puis on extrait par 3 fois 50 cm3 d'éther éthylique. Les phases organiques sont réunies et séchées sur sulfate de sodium. Après filtration et évaporation du solvant, on obtient 7,47 g d'une huile légèrement jaune constituée d'un mélange 55/45 de:

$(CH_3)_2C=CH-CH_2CH_2-C(=CH_2)-CH_2-CH_2-CCl(COOCH_3)COCH_3$
et
$(CH_3)_2C=CH-CH_2CH_2-C(CH_3)=CHCH_2-CCl(COOCH_3)COCH_3$ .

L'analyse par chromatographie en phase gazeuse, avec étalon interne, montre que:
- le taux de transformation est de 98%
- le rendement est de 97%.

*Exemple 3*

Dans un ballon de 100 cm3, on introduit, sous atmosphère d'argon, 50 cm3 de N-méthylpyrrolidone et 4,8 g (0,0190 mole) d'un mélange 55/45 de:

$(CH_3)_2C=CH-CH_2CH_2-C(=CH_2)-CH_2CH_2-CH(COOCH_3)COCH_3$
et
$(CH_3)_2C=CH-CH_2CH_2-C(CH_3)=CH-CH_2-CH(COOCH_3)COCH_3$

puis on ajoute 1,10 g (0,01964 mole) de potasse broyée et 5 g (0,021 mole) d'hexachloroéthane. On agite pendant 40 minutes à 25°C. Le mélange réactionnel est versé dans 100 cm3 d'eau puis on extrait par 3 fois 50 cm3 d'éther éthylique. Les phases organiques sont réunies et séchées sur sulfate de sodium. Après filtration et évaporation du solvant, on obtient 8,2 g d'une huile légèrement jaune qui contient un mélange 55/45 de:

$(CH_3)_2C=CH-CH_2CH_2-C(=CH_2)CH_2CH_2-CCl(COOCH_3)COCH_3$
et
$(CH_3)_2C=CH-CH_2CH_2-C(CH_3)=CH-CH_2-CCl(COOCH_3)COCH_3$

L'analyse par chromaographie en phase gazeuse, avec étalon interne, montre que:
- le taux de transformation est voisin de 98-99%
- le rendement est de 90%.

*Exemple 4*

Dans un ballon de 100 cm3, on introduit, sous atmosphère d'argon, 15 cm3 d'hexane, 5 cm3 de N-méthylpyrrolidone, 0,56 g (0,01 mole) de potasse puis 2,52 g (0,01 mole) d'un mélange 55/45 de:

$(CH_3)_2C=CH-CH_2CH_2-C(=CH_2)CH_2CH_2-CH(COOCH_3)COCH_3$
et
$(CH_3)_2C=CH-CH_2CH_2-C(CH_3)=CH-CH_2-CH(COOCH_3)COCH_3$

On ajoute alors 2,36 g (0,01 mole) d'hexachloroéthane. On agite pendant 15 heures à 25°C. Le mélange réactionnel est versé dans 100 cm3 d'eau puis on extrait par 3 fois 50 cm3 d'éther éthylique. Les phases organiques réunies sont séchées sur sulfate de magnésium. Après filtration et évaporation du solvant, on obtient 3,9 g d'une huile légèrement jaune constituée d'un mélange 55/45 de:

$(CH_3)_2C=CH-CH_2CH_2-C(=CH_2)-CH_2CH_2-CCl(COOCH_3)COCH_3$
et
$(CH_3)_2C=CH-CH_2CH_2-C(CH_3)=CH-CH_2-CCl(COOCH_3)COCH_3$.

Le rendement en produit isolé après une flash distillation est de 70%.

*Exemple 5*

Dans un ballon de 100 cm3, on introduit, sous atmosphère d'argon, 20 cm3 de N-méthylpyrrolidone et 2,52 g (0,01 mole) d'un mélange 55/45 de:

$(CH_3)_2C=CH-CH_2CH_2-C(=CH_2)CH_2CH_2-CH(COOCH_3)COCH_3$
et
$(CH_3)_2C=CH-CH_2CH_2-C(CH_3)=CH-CH_2-CH(COOCH_3)COCH_3$

puis on ajoute 1,5 g (0,0108 mole) de carbonate de potassium et 2,36 g (0,01 mole) d'hexachloroéthane. On agite pendant 24 heures à 25°C. Le mélange réactionnel est versé dans 100 cm3 d'eau puis on extrait par 3 fois 50 cm3 d'éther éthylique. Les phases organiques réunies sont séchées sur sulfate de magnésium. Après filtration et évaporation du solvant, on obtient 3 g d'une huile légèrement jaune constituée d'un mélange 55/45 de:

$(CH_3)_2C=CH-CH_2CH_2-C(=CH_2)-CH_2-CH_2-CCl(COOCH_3)COCH_3$
et
$(CH_3)_2C=CH-CH_2CH_2-C(CH_3)=CH-CH_2-CCl(COOCH_3)COCH_3$.

Le taux de transformation est de 80%.
Le rendement en produit isolé est de 73%.

*Exemple 6*

Dans un ballon de 100 cm3, on introduit, sous atmosphère d'argon, 20 cm3 de tétrahydrofuranne et 2,52 g (0,01 mole) d'un mélange 55/45 de:

$(CH_3)_2C=CH-CH_2CH_2-C(=CH_2)CH_2CH_2-CH(COOCH_3)COCH_3$
et
$(CH_3)_2C=CH-CH_2CH_2-C(CH_3)=CH-CH_2-CH(COOCH_3)COCH_3$

puis on ajoute 0,56 g (0,01 mole) de potasse et 2,5 g (0,0105 mole) d'hexachloroéthane. On agite pendant 10 heures à 25°C. Le mélange réactionnel est versé dans 100 cm3 d'eau puis on extrait par 3 fois 50 cm3 d'éther éthylique. Les phases organiques réunies sont séchées sur sulfate de magnésium. Après filtration et évaporation du solvant, on obtient une huile légèrement jaune constituée d'un mélange 55/45 de:

$(CH_3)_2C=CH-CH_2CH_2-C(=CH_2)-CH_2CH_2-CCl(COOCH_3)COCH_3$
et
$(CH_3)_2C=CH-CH_2CH_2-C(CH_3)=CH-CH_2-CCl(COOCH_3)COCH_3$.

Le rendement en produit isolé est de 70%.

*Exemple 7*

Dans un ballon de 100 cm3, on introduit, sous atmosphère d'argon, 20 cm3 de méthanol et, par petites fractions 0,23 g (0,01 atome-gramme) de sodium. On refroidit à −50°C puis on ajoute 2,52 g (0,01 mole) d'un mélange 55/45 de:

$(CH_3)_2C=CH-CH_2CH_2-C(=CH_2)CH_2CH_2-CH(COOCH_3)COCH_3$
et
$(CH_3)_2C=CH-CH_2CH_2-C(CH_3)=CH-CH_2-CH(COOCH_3)COCH_3$

et 1,33 g (0,01 mole de N-chlorsuccinimide. On agite pendant 40 minutes à −50°C. Après évaporation du méthanol, le mélange réactionnel est versé dans 100 cm3 d'eau puis on extrait par 3 fois 50 cm3 d'éther éthylique. Les phases organiques réunies sont séchées sur sulfate de magnésium. Après filtration et évaporation du solvant, on obtient, après une flash-distillation, 2,5 g d'une huile légèrement jaune constituée d'un mélange 55/45 de:

$(CH_3)_2C=CH-CH_2CH_2-C(=CH_3)CH_2CH_2-CCl(COOCH_3)COCH_3$
et
$(CH_3)_2C=CH-CH_2CH_2-C(CH_3)=CH-CH_2-CCl(COOCH_3)COCH_3$.

Le rendement est de 87%.

*Exemple 8*

Dans un réacteur de 1200 cm3, on introduit, sous atmosphère d'argon 500 cm3 d'hexane et 4,04 g (0,103 mole) d'amidure de sodium. On refroidit à 5°C puis on ajoute lentement, en 1 heure 40 minutes, une solution de 24,4 g (0,097 mole) d'un mélange 55/45 de:

$(CH_3)_2C=CH-CH_2CH_2-C(=CH_2)CH_2CH_2-CH(COOCH_3)COCH_3$
et
$(CH_3)_2C=CH-CH_2CH_2-C(CH_3)=CH-CH_2-CH(COOCH_3)COCH_3$

dans 100 cm3 d'hexane. On agite ensuite pendant 5 heures à 5°C; il se dégage 95% de la quantité théorique d'ammoniac. On ajoute ensuite, en 45 minutes, une solution de 14,5 g (0,107 mole) de chlorure de sulfuryle dans 50 cm3 d'hexane. On agite pendant 1 heure 30 minutes: on observe la formation d'un précipité blanc. Après filtration et évaporation du solvant, on obtient 31,2 g d'une huile légèrement jaune constituée d'un mélange 55/45 de:

$(CH_3)_2C=CH-CH_2CH_2-C(=CH_3)CH_2CH_2-CCl(COOCH_3)COCH_3$
et
$(CH_3)_2C=CH-CH_2CH_2-C(CH_3)=CH-CH_2-CCl(COOCH_3)COCH_3$.

Un dosage par chromatographie en phase gazeuse, avec étalon interne, montre que:
- le taux de transformation est de 93,5%
- le rendement est de 90,2%.

*Exemple 9*

Dans un réacteur de 250 cm3, on introduit, sous atmosphère d'argon, 80 cm3 de toluène et 0,76 g (0,0194 mole) d'amidure de sodium. On refroidit à une température comprise entre 5 et 10°C puis on ajoute une solution de 4,96 g (0,0197 mole) d'un mélange 55/45 de:

$(CH_3)_2C=CH-CH_2CH_2-C(=CH_2)CH_2CH_2-CH(COOCH_3)COCH_3$
et
$(CH_3)_2C=CH-CH_2CH_2-C(CH_3)=CH-CH_2-CH(COOCH_3)COCH_3$

dans 20 cm3 de toluène. On laisse réagir pendant 2 heures à 25°C puis on ajoute une solution de 2,89 g (0,0215 mole) de chlorure de sulfuryle dans 10 cm3 de toluène. On agite pendant 1 heure. Après filtration et évaporation du solvant, on obtient 5,63 g d'une huile légèrement jaune constituée d'un mélange 55/45 de:

$(CH_3)_2C=CH-CH_2CH_2-C(=CH_2)CH_2CH_2-CCl(COOCH_3)COCH_3$
et
$(CH_3)_2C=CH-CH_2CH_2-C(CH_3)=CH-CH_2-CCl(COOCH_3)COCH_3$.

L'analyse par chromatographie en phase gazeuse, avec étalon interne, montre que le rendement est de 66,8%.

*Exemple 10*

Dans un ballon de 100 cm3, on introduit, sous

atmosphère d'argon, 20 cm3 de méthanol et, par petites fractions, 0,23 g (0,01 atome-gramme) de sodium. On refroidit à -50°C puis on ajoute 2,52 g (0,01 mole) d'un mélange 55/45 de:

$(CH_3)_2C=CH-CH_2CH_2-C(=CH_2)CH_2CH_2-CH(COOCH_3)COCH_3$
et
$(CH_3)_2C=CH-CH_2CH_2-C(CH_3)=CH-CH_2-CH(COOCH_3)COCH_3$

On ajoute ensuite, à une température de -50°C, 1,34 g (0,01 mole) de chlorure de sulfuryle. On agite pendant 30 minutes à -50°C. Après avoir laissé la température remonter au voisinage de 20°C, le mélange réactionnel est versé dans 100 cm3 d'eau puis on extrait par 3 fois 50 cm3 d'éther éthylique. Les phases organiques réunies sont séchées sur sulfate de magnésium. Après filtration et évaporation du solvant, on obtient 4,7 g d'une huile légèrement jaune contenant un mélange 55/45 de:

$(CH_3)_2C=CH-CH_2CH_2-C(=CH_2)CH_2CH_2-CCl(COOCH_3)COCH_3$
et
$(CH_3)_2C=CH-CH_2CH_2-C(CH_3)=CH-CH_2-CCl(COOCH_3)COCH_3$.

Le rendement en produit isolé est de 65%.

*Exemple 11*

Dans un ballon de 100 cm3, on introduit, sous atmosphère d'argon, 20 cm3 de méthanol et 0,23 g (0,01 atome-gramme) de sodium. On refroidit à 0°C puis on ajoute 2,52 g (0,01 mole) d'un mélange 55/45 de:

$(CH_3)_2C=CH-CH_2CH_2-C(=CH_2)-CH_2CH_2-CH(COOCH_3)COCH_3$
et
$(CH_3)_2C=CH-CH_2CH_2-C(CH_3)=CH-CH_2-CH(COOCH_3)COCH_3$

et 1,33 g (0,01 mole) de N-chlorosuccinimide. On agite pendant 40 minutes à 0°C. Le mélange réactionnel est versé dans 100 cm3 d'eau puis on extrait par 3 fois 50 cm3 d'éther. Les phases organiques réunies sont séchées sur sulfate de magnésium. Après filtration et évaporation du solvant, on obtient 2,3 g d'une huile jaune qui, d'après l'analyse par chromatographie en phase gazeuse, contient:

- 6,5% de produit de départ
- 41,6% d'un mélange 55/45 de:

$(CH_3)_2C=CH-CH_2CH_2-C(=CH_2)-CH_2CH_2-CHCl-COOCH_3$
et
$(CH_3)_2C=CH-CH_2CH_2-C(CH_3)=CH-CH_2-CHCl-COOCH_3$

- 51,8% d'un mélange 55/45 de:

$(CH_3)_2C=CH-CH_2CH_2-C(=CH_2)-CH_2CH_2-CCl(COOCH_3)COCH_3$
et
$(CH_3)_2C=CH-CH_2CH_2-C(CH_3)=CH-CH_2-CCl(COOCH_3)COCH_3$

*Exemple 12*

Dans un ballon de 100 cm3, on introduit, sous atmosphère d'argon, 0,5 g (0,01 mole) d'hydrure de sodium à 50% dans l'huile minérale. On lave par 3 fois 20 cm3 de pentane sec. On ajoute alors 20 cm3 de tétrahydrofuranne et 2,52 g (0,01 mole) d'un mélange 55/45 de:

$(CH_3)_2C=CH-CH_2CH_2-C(=CH_2)-CH_2CH_2-CH(COOCH_3)COCH_3$
et
$(CH_3)_2C=CH-CH_2CH_2-C(CH_3)=CH-CH_2-CH(COOCH_3)COCH_3$.

On laisse réagir pendant 5 heures à 25°C puis on ajoute ensuite lenemtenent une solution de 1,34 (0,01 mole) de chlorure de sulfuryle dans 10 cm3 de tétrahydrofuranne. On agite pendant 3 heures à 25°C. Le mélange réactionnel est versé dans 100 cm3 d'eau puis on extrait par 3 fois 50 cm3 d'éther éthylique. Les phases organiques réunies sont séchées sur sulfate de magnésium. Après filtration et évaporation du solvant, on obtient 3,0 g d'une huile jaune qui, d'après l'analyse par chromatographie en phase gazeuse, contient 62% d'un mélange 55/45 de:

$(CH_3)_2C=CH-CH_2CH_2-C(=CH_3)-CH_2CH_2-CCl(COOCH_3)COCH_3$
et
$(CH_3)_2C=CH-CH_2CH_2-C(CH_3)=CH-CH_2-CCl(COOCH_3)COCH_3$.

D'après l'analyse par chromatographie en phase gazeuse, le rendement est de 75%.

*Exemple 13*

Dans un ballon de 100 cm3, on introduit, sous atmosphère d'argon, 40 cm3 d'éther sec, 0,236 g (0,01029 atome-gramme) de sodium et quelques cristaux de chlorure ferrique. On ajoute ensuite 2,42 g (0,00961 mole) d'un mélange 55/45 de:

$(CH_3)_2C=CH-CH_2CH_2-C(=CH_2)-CH_2CH_2-CH(COOCH_3)COCH_3$
et
$(CH_3)_2C=CH-CH_2CH_2-C(CH_3)=CH-CH_2-CH(COOCH_3)COCH_3$

On laisse réagir pendant 4 heures à 25°C. Après refroidissement à 0°C, on ajoute une solution de 1,30 g (0,00963 mole) de chlorure de sulfuryle dans 5 cm3 d'éther. On agite pendant 30 minutes à 0°C. Le mélange réactionnel est versé dans 100 cm3 d'eau et on extrait par 3 fois 50 cm3 d'éther éthylique. Les phases organiques réunies sont séchées sur sulfate de magnésium. Après filtration et évaporation du solvant, on obtient 2,71 g d'une huile jaune constituée d'un mélange 55/45 de:

$(CH_3)_2C=CH-CH_2CH_2-C(=CH_3)CH_2CH_2-CCl(COOCH_3)COCH_3$
et
$(CH_3)_2C=CH-CH_2CH_2-C(CH_3)=CH-CH_2-CCl(COOCH_3)COCH_3$.

L'analyse par chromatographie en phase gazeuse, avec étalon interne, montre que:

- le taux de transformation est de 74,8%
- le rendement en produits chlorés est de 67%.

*Exemple 14*

Dans un ballon de 500 cm3, on introduit, sous atmosphère d'argon, 200 cm3 de diméthylformamide, 16,4 g (0,118 mole) de carbonate de potassium, 25,2 g (0,01 mole) d'un mélange 55/45 de:

$(CH_3)_2C=CH-CH_2CH_2-C(=CH_2)-CH_2CH_2-CH(COOCH_3)COCH_3$
et
$(CH_3)_2C=CH-CH_2CH_2-C(CH_3)=CH-CH_2-CH(COOCH_3)COCH_3$

et 14 g (0,104 mole) de N-chlorosuccinimide. On laisse réagir pendant 16 heures à 25°C. Le mélange réactionnel est versé dans 100 cm3 d'eau et on extrait par 3 fois 50 cm3 d'éther éthylique. Les phases organiques réunies sont séchées sur sulfate de magnésium. Après filtration et évaporation du solvant, on obtient 27 g d'une huile légèrement jaune qui contient 75% d'un mélange 55/45 de:

$(CH_3)_2C=CH-CH_2CH_2-C(=CH_2)-CH_2CH_2-CCl(COOCH_3)COCH_3$
et
$(CH_3)_2C=CH-CH_2CH_2-C(CH_3)=CH-CH_2-CCl(COOCH_3)COCH_3$.

*Exemple 15*

Dans un ballon de 500 cm3, on introduit, sous atmosphère d'argon, 150 cm3 de diméthylformamide, 27,6 g (0,2 mole) de carbonate de potassium et 38 g (0,15 mole) d'un mélange 55/45 de:

$(CH_3)_2C=CH-CH_2CH_2-C(=CH_2)-CH_2CH_2-CH(COOCH_3)COCH_3$
et
$(CH_3)_2C=CH-CH_2CH_2-C(CH_3)=CH-CH_2-CH(COOCH_3)COCH_3$

puis on ajoute 42 g (0,177 mole) d'hexachloroéthane. On agite pendant 15 heures à 25°C. On ajoute 200 cm3 d'eau puis on extrait par 3 fois 50 cm3 d'éther éthylique. Les phases organiques réunies sont séchées sur sulfate de magnésium. Après filtration et évaporation du solvant, on obtient 60 g d'une huile qui, par distillation à 150°C sous pression réduite (1 mm de mercure, 0,13 kPa), fournit une huile qui contient 94% d'un mélange 55/45 de:

$(CH_3)_2C=CH-CH_2CH_2-C(=CH_2)CH_2CH_2-CCl(COOCH_3)COCH_3$
et
$(CH_3)_2C=CH-CH_2CH_2-C(CH_3)=CH-CH_2-CCl(COOCH_3)COCH_3$.

Le rendement en produit isolé est de 81%.

La structure du produit obtenu est confirmée par le spectre de résonance magnétique nucléaire du proton.

*Exemple 16*

Dans un ballon de 200 cm3, on introduit, sous atmosphère d'argon, 20 cm3 de méthyltertiobutyléther, 1,5 g (0,0108 mole) de carbonate de potassium et 2,52 g (0,01 mole) d'un mélange 55/45 de:

$(CH_3)_2C=CH-CH_2CH_2-C(=CH_2)-CH_2CH_2-CH(COOCH_3)COCH_3$
et
$(CH_3)_2C=CH-CH_2CH_2-C(CH_3)=CH-CH_2-CH(COOCH_3)COCH_3$

puis on ajoute 1,33 g (0,01 mole) de N-chlorosuccinimide. On agite pendant 15 heures à 25°C. On ajoute 100 cm3 d'eau puis on extrait par 3 fois 50 cm3 d'éther éthylique. Les phases organiques réunies sont séchées sur sulfate de magnésium. Après filtration et évaporation du solvant, on obtient 2,3 g d'une huile incolore qui contient 91% d'un mélange 55/45 de:

$(CH_3)_2C=CH-CH_2CH_2-C(=CH_2)CH_2CH_2-CCl(COOCH_3)COCH_3$
et
$(CH_3)_2C=CH-CH_2CH_2-C(CH_3)=CH-CH_2-CCl(COOCH_3)COCH_3$.

Le rendement en produit isolé est de 73%.

*Exemple 17*

Dans un ballon de 100 cm3, on introduit, sous atmosphère d'argon, 20 cm3 de N-méthylpyrrolidone, 2,8 g (0,0202 mole) de carbonate de potassium et 3,68 g(0,02 mole) d'un mélange 55/45 de:

$CH_2=C(CH_3)-CH_2CH_2-CH(COOCH_3)COCH_3$
et
$(CH_3)_2C=CH-CH_2-CH(COOCH_3)COCH_3$

puis on ajoute 5 g (0,0211 mole) d'hexachloroéthane. On agite pendant 15 heures à 25°C. On ajoute ensuite 100 cm3 d'eau puis on extrait par 3 fois 50 cm3 d'éther éthylique. Les phases organiques réunies sont séchées sur sulfate de magnésium. Après filtration et évaporation du solvant, on obtient 6,4 g d'une huile légèrement jaune qui contient 16% de produit de départ et 41% d'un mélange 55/45 de:

$CH_2=C(CH_3)-CH_2CH_2-CCl(COOCH_3)COCH_3$
et
$(CH_3)_2C=CH-CH_2-CCl(COOCH_3)COCH_3$.

Le rendement par rapport au produit de départ ayant réagi est de 83%.

*Exemple 18*

Dans un ballon de 250 cm3, on introduit, sous atmosphère d'argon, 80 cm3 d'acétone, 8,28 g de carbonate de potassium (0,05993 mole), 13,17 g d'hexachloroéthane (0,05565 mole) et 10,86 g (0,04312 mole) d'un mélange 55/45 de:

$(CH_3)_2C=CH-CH_2CH_2-C(=CH_2)-CH_2CH_2-CH(COOCH_3)COCH_3$
et
$(CH_3)_2C=CH-CH_2CH_2-C(CH_3)=CH-CH_2-CH(COOCH_3)COCH_3$

puis on agite pendant 7 heures à la température de reflux du mélange réactionnel. Le mélange réactionnel est versé dans 100 cm3 d'eau légèrement acidulée puis on extrait par 3 fois 50 cm3 d'éther éthylique. Les phases organiques réunies sont séchées sur sulfate de magnésium. Après évaporation du solvant, on obtient 15,45 g d'un liquide jaune orangé qui contient un mélange de:

$(CH_3)_2C=CH-CH_2CH_2-C(=CH_2)-CH_2CH_2-CCl(COOCH_3)COCH_3$
et
$(CH_3)_2C=CH-CH_2CH_2-C(CH_3)=CH-CH_2-CCl(COOCH_3)COCH_3$.

D'après l'analyse par chromatographie en phase gazeuse, avec étalon interne, le rendement est de 91,5%.

*Exemple 19*

Dans un ballon de 100 cm3, on introduit, sous atmosphère d'argon, 20 cm3 de diméthylformamide, 2,5 g (0,0105 mole) d'hexachloroéthane et 2,52 g (0,01 mole) d'un mélange 55/45 de:

$(CH_3)_2C=CH-CH_2CH_2-C(=CH_2)-CH_2CH_2-CH(COOCH_3)COCH_3$
et
$(CH_3)_2C=CH-CH_2CH_2-C(CH_3)=CH-CH_2-CH(COOCH_3)COCH_3$

puis on ajoute 2 g (0,0131 mole) de 1,8-diazabicyclo [5,4,0] undec-7-ène (DBU). On agite pendant 4 heures à 25°C. Le mélange réactionnel est versé dans 100 cm3 d'eau puis on extrait par 3 fois 50 cm3 d'éther éthylique. Les phases organiques sont séchées sur sulfate de magnésium. Après filtration et évaporation du solvant, on obtient 4,12 g d'une huile légèrement jaune qui, après une flash-distillation, conduit à 2,41 g d'une huile jaune clair contenant 87% d'un mélange 55/45 de:

$(CH_3)_2C=CH-CH_2CH_2-C(=CH_2)CH_2CH_2-CCl(COOCH_3)COCH_3$
et
$(CH_3)_2C=CH-CH_2CH_2-C(CH_3)=CH_2CH_2-CCl(COOCH_3)COCH_3$.

Le rendement en produit isolé est de 73%.

*Exemple 20*

Dans un ballon de 100 cm3, on introduit, sous atmosphère d'argon, 7,11 g (0,03 mole) d'hexachloroéthane, 0,342 g (0,001 mole) d'hydrogénosulfate de tétrabutylammonium et 2,52 g (0,01 mole) d'un mélange 55/45 de:

$(CH_3)_2C=CH\text{-}CH_2\text{-}C(=CH_2)\text{-}CH_2CH_2\text{-}CH(COCH_3)COOCH_3$
et
$(CH_3)_2C=CH\text{-}CH_2\text{-}C(CH_3)=CH\text{-}CH_2\text{-}CH(COCH_3)COOCH_3$

en solution dans 50 cm3 de chlorure de méthylène. On ajoute ensuite 5 cm3 d'une solution aqueuse de soude à 50% en poids on agite vigoureusement pendant 2 heures à 25°C. Après extraction du mélange réactionnel, on obtient, après chromatographie, 1,62 g d'un mélange 55/45 de:

$(CH_3)_2C=CH\text{-}CH_2\text{-}C(=CH_2)CH_2CH_2\text{-}CCl(COCH_3)COOCH_3$
et
$(CH_3)_2C=CH\text{-}CH_2\text{-}C(CH_3)=CH\text{-}CH_2\text{-}CCl(COCH_3)COOCH_3$.

Le rendement est de 56,5%.

*Exemple 21*

Dans un ballon de 100 cm3, on introduit, sous atmosphère d'argon, 3,08 g d'hexachloroéthane, 2,07 g de carbonate de potassium et 0,34 g d'hydrogénosulfate de tétrabutylammonium dans 20 cm3 de chlorure de méthylène. On ajoute ensuite 2,52 g (10 m.moles) d'un mélange 55/45 de:

$(CH_3)_2C=CH\text{-}CH_2\text{-}C(=CH_2)\text{-}CH_2CH_2\text{-}CH(COCH_3)COOCH_3$
et
$(CH_3)_2C=CH\text{-}CH_2\text{-}C(CH_3)=CH\text{-}CH_2\text{-}CH(COCH_3)COOCH_3$.

On chauffe le mélange réactionnel pendant 5 heures au reflux.

On obtient, avec un rendement de 20%, un mélange 55/45 de:

$(CH_3)_2C=CH\text{-}CH_2\text{-}C(=CH_2)CH_2CH_2\text{-}CCl(COCH_3)COOCH_3$
et
$(CH_3)_2C=CH\text{-}CH_2\text{-}C(CH_3)=CH\text{-}CH_2\text{-}CCl(COCH_3)COOCH_3$.

*Exemple 22*

Dans un ballon de 100 cm3, on introduit, sous atmosphère d'argon, 11,04 g (0,08 mole) de carbonate de potassium, 4,96 g (0,021 mole) d'hexachloroéthane et 20 cm3 d'acétonitrile. On ajoute 5,04 (0,02 mole) d'un mélange 55/45 de:

$(CH_3)_2C=CH\text{-}CH_2\text{-}C(=CH_2)\text{-}CH_2CH_2\text{-}CH(COCH_3)COOCH_3$
et
$(CH_3)_2C=CH\text{-}CH_2\text{-}C(CH_3)=CH\text{-}CH_2\text{-}CH(COCH_3)COOCH_3$.

On chauffe le mélange réactionnel au reflux pedant 3 heures. Après refroidissement, le mélange réactionnel est versé dans 100 cm3 d'eau puis on extrait par 3 fois 50 cm3 d'éther éthylique. Les phases organiques sont lavées à l'eau puis séchées sur sulfate de magnésium. Après filtration et évaporation du solvant, on obtient, avec un rendement de 95%, un mélange 55/45 de:

$(CH_3)_2C=CH\text{-}CH_2\text{-}C(=CH_2)CH_2CH_2\text{-}CCl(COCH_3)COOCH_3$
et
$(CH_3)_2C=CH\text{-}CH_2\text{-}C(CH_3)=CH\text{-}CH_2\text{-}CCl(COCH_3)COOCH_3$

sous forme d'une huile orange.

*Exemple 23*

Dans un ballon de 100 cm3, on introduit 2,37 g (0,01 mole) d'hexachloroéthane, 1,38 g (0,01 mole) de carbonate de potassium et 10 cm3 de nitrobenzène. On ajoute 1,26 g (0,005 mole) d'un mélange 55/45 de:

$(CH_3)_2C=CH\text{-}CH_2\text{-}C(=CH_2)\text{-}CH_2CH_2\text{-}CH(COCH_3)COOCH_3$
et
$(CH_3)_2C=CH\text{-}CH_2\text{-}C(CH_3)=CH\text{-}CH_2\text{-}CH(COCH_3)COOCH_3$.

Le mélange hétérogène est agité pendant 5 heures à 110°C.

Après extraction, on obtient, avec un rendement de 78%, un mélange 55/45 de:

$(CH_3)_2C=CH\text{-}CH_2\text{-}C(=CH_2)CH_2CH_2\text{-}CCl(COCH_3)COOCH_3$
et
$(CH_3)_2C=CH\text{-}CH_2\text{-}C(CH_3)=CH\text{-}CH_2\text{-}CCl(COCH_3)COOCH_3$.

**Revendications**

1. Procédé de préparation de composés insaturés chlorés en α de deux groupements électroattracteurs en position β de formule générale (Ia) ou (Ib) dans laquelle n représente un nombre entier compris entre 1 et 10 inclusivement, X et Y, identiques ou différents, représentent chacun un groupement électroattracteur choisi parmi les raicaux -CHO, $-COR_1$, $-COOR_2$, $-CONR_3R_4$, -CN, $-SO_2R_5$ et $-NO_2$ dans lesquels $R_1$, $R_2$, $R_3$, $R_4$ et $_5$ représentent des radicaux alcoyles contenant 1 à 4 atomes de carbone, $R_5$ pouvant en outre représenter un radical phényle éventuellement substitué par un radical méthyle, caractérisé en ce que l'on fait réagir un agent d'halogénation capable de fournir un cation $Cl^+$ sur un produit de formule générale (IIa) ou (IIb) dans laquelle n, X et Y sont définis comme précédemment, anionisé au moyen d'une base.

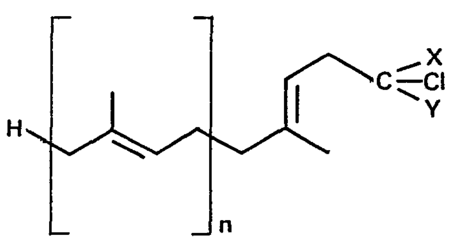

(Ia)

ou

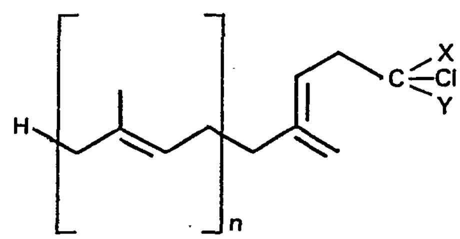

(Ib)

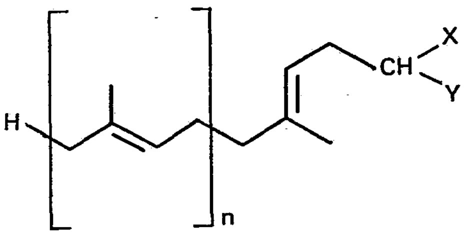

(IIa)

ou

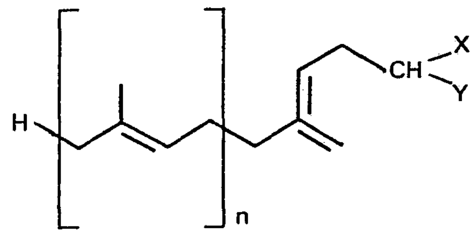

(IIb)

2. Procédé selon la revendication 1, caractérisé en ce que l'agent d'halogénation est choisi parmi le chlore moléculaire, le chlorure de sulfuryle, le N-chlorosuccinimide et l'hexachloroéthane.

3. Procédé selon la revendication 1, caractérisé en ce que l'agent d'anionisation est choisi parmi les hydrures, les amidures, les hydroxydes et les carbonates de métal alcalin ou alcalinoterreux et les amines non quaternaires.

4. Procédé selon l'une des revendications 1, 2 ou 3, caractérisé en ce que l'on opère dans un solvant organique choisi parmi les hydrocarbures aliphatiques, les hydrocarbures aromatiques, les éthers, les cétones, les amides tertiaires, les nitriles, les solvants nitrés et, lorsque l'on opère en présence d'un catalyseur de transfert de phase, les solvants chlorés.

5. Procédé selon l'une des revendications 1, 2, 3 ou 4, caractérisé en ce que l'on opère à une température comprise entre 0 et 120°C.

6. Procédé selon la revendication 1, caractérisé en ce que l'agent d'anionisation est un alcoolate de métal alcalin dans l'alcool correspondant.

7. Procédé selon la revendication 6, caractérisé en ce que l'on opère à une température inférieure à 0°C.

8. Procédé selon la revendication 6, caractérisé en ce que l'on opère à une température inférieure à -30°C.

9. Procédé selon la revendication 1, caractérisé en ce que l'on ajoute au produit de formule générale (IIa) ou (IIb) en solution dans un solvant organique l'agent d'anionisation et l'agent d'halogénation.

10. Procédé selon la revendication 1, caractérisé en ce que l'on effectue d'abord l'anionisation du produit de formule générale (IIa) ou (IIb) puis ajoute l'agent d'halogénation.

**Patentansprüche**

1. Verfahren zur Herstellung von in α-Stellung zu zwei in β-Stellung befindlichen elektronenanziehenden Gruppen chlorierten ungesättigten Verbindungen der allgemeinen Formel (Ia) oder (Ib), in welcher n eine ganze Zahl zwischen 1 und 10 inklusive darstellt und X und Y, die gleich oder voneinander verschieden sind, jeweils eine elektronenanziehende Gruppe, ausgewählt aus den Resten -CHO, $-COR_1$, $-COOR_2$, $-CONR_3R_4$, -CN, $-SO_2R_5$ und $-NO_2$, darstellt, worin $R_1$, $R_2$, $R_3$, $R_4$ und $R_5$ für Alkylreste mit 1 bis 4 Kohlenstoffatomen stehen und $R_5$ ausserdem einen Phenylrest, gegebenenfalls substituiert durch einen Methylrest, darstellen kann, dadurch gekennzeichnet, dass man ein Halogenierungsmittel, das ein Kation $Cl^+$ zu liefern vermag, mit einer Verbindung der allgemeinen Formel (IIa) oder (IIb) reagieren lässt, worin n, X und Y die obige Bedeutung haben und das durch eine Base anionisiert ist.

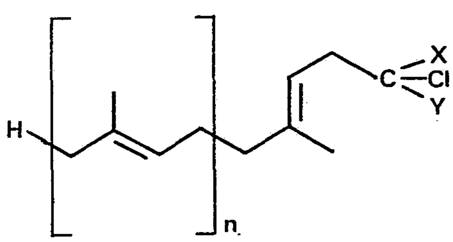

(Ia)

oder

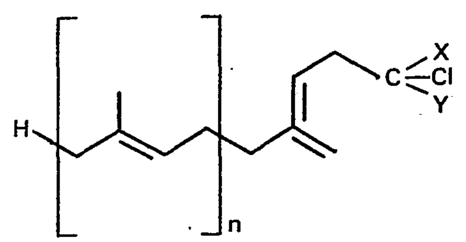

(Ib)

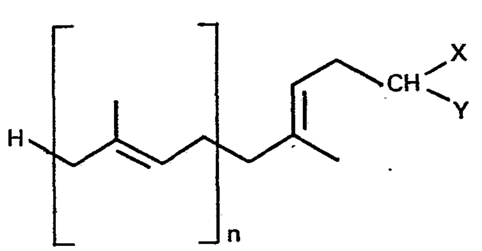

(IIa)

oder

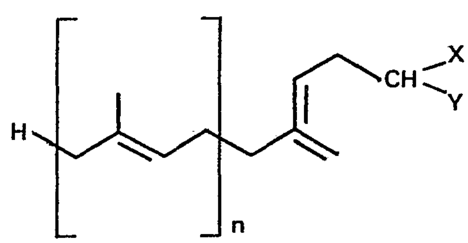

(IIb)

2. Verfahren nach Anspruch 1, dadurch gekennzeichnet, dass das Halogenierungsmittel ausgewählt wird aus molekularem Chlor, Sulfurylchlorid, N-Chlorsuccinimid und Hexachloräthan.

3. Verfahren nach Anspruch 1, dadurch gekennzeichnet, dass das Anionisierungsmittel ausgewählt wird aus den Alkali- oder Erdalkalimetallhydriden, -amiden, -hydroxiden und carbonaten und den nicht quaternisierbaren Aminen.

4. Verfahren nach einem der Ansprüche 1, 2 oder 3, dadurch gekennzeichnet, dass man in einem organischen Lösungsmittel, ausgewählt aus den aliphatischen Kohlenwasserstoffen, den aromatischen Kohlenwasserstoffen, den Äthern, den Ketonen, den tertiären Amiden, den Nitrilen, den nitrierten Lösungsmitteln und, wenn man in Anwesenheit eines Phasenübertragungskatalysators arbeitet, den chlorierten Lösungsmitteln, arbeitet.

5. Verfahren nach einem der Ansprüche 1, 2, 3 oder 4, dadurch gekennzeichnet, dass man bei einer Temperatur zwischen 0 und 120°C arbeitet.

6. Verfahren nach Anspruch 1, dadurch gekennzeichnet, dass das Anionisierungsmittel ein Alkalimetallalkoholat in dem entsprechenden Alkohol ist.

7. Verfahren nach Anspruch 6, dadurch gekennzeichnet, dass man bei einer Temperatur unterhalb 0°C arbeitet.

8. Verfahren nach Anspruch 6, dadurch gekennzeichnet, dass man bei einer Temperatur unterhalb −30°C arbeitet.

9. Verfahren nach Anspruch 1, dadurch gekennzeichnet, dass man das Anionisierungsmittel und das Halogenierungsmittel der in einem organischen Lösungsmittel gelösten Verbindung der allgemeinen Formel (IIa) oder (IIb) zusetzt.

10. Verfahren nach Anspruch 1, dadurch gekennzeichnet, dass man zuerst die Anionisierung der Verbindung der allgemeinen Formel (IIa) oder (IIb) ausführt und dann das Halogenierungsmittel zusetzt.

**Claims**

1. Process for the preparation of unsaturated compounds chlorinated in the α position with respect to two electron-withdrawing groups in the β position of general formula (Ia) or (Ib) in which n denotes an integer from 1 to 10 inclusive, X and Y, which are identical or different, each denote an electron-withdrawing group chosen from -CHO, $-COR_1$, $-COOR_2$, $-CONR_3R_4$, -CN, $-SO_2R_5$ and $-NO_2$ radicals in which $R_1$, $R_2$, $R_3$, $R_4$ and $R_5$ denote alkyl radical containing 1 to 4 carbon atoms, it being additionally possible for $R_5$ to denote a phenyl radical optionally substituted by a methyl radical, characterized in that a halogenating agent capable of providing a $Cl^+$ cation is reacted with a product of general formula (IIa) or (IIb) in which n, X and Y are defined as previously, which is anionized by means of a base.

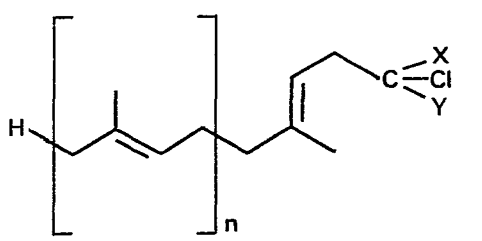

(Ia)

or

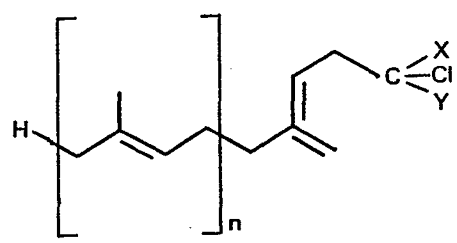

(Ib)

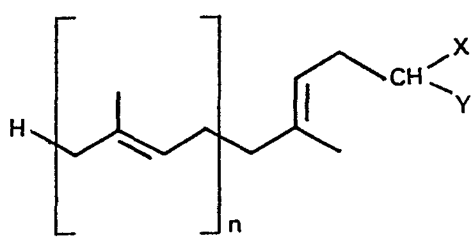

(IIa)

or

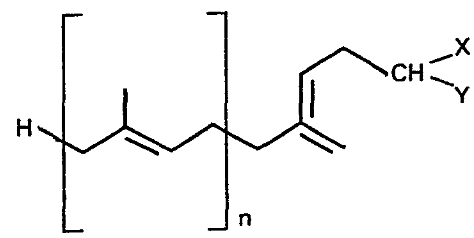

(IIb)

2. Process according to claim 1, characterized in that the halogenating agent is chosen from molecular chlorine, sulphuryl chloride, N-chlorosuccinimide and hexachloroethane.

3. Process according to claim 1, characterized in that that the anionizing agent is chosen from alkali metal or alkaline-earth metal hydrides, amides, hydroxides and carbonates and nonquaternizable amines.

4. Process according to one of claims 1, 2 or 3, characterized in that the operation is performed in an organic solvent chosen from aliphatic hydrocarbons, aromatic hydrocarbons, ethers, ketones, tertiary amides, nitriles, nitro solvents and, when the operation is performed in the presence of a phase transfer catalyst, chlorinated solvents.

5. Process according to one of claims 1, 2, 3 or 4, characterized in that the operation is performed at a temperature between 0 and 120°C.

6. Process according to claim 1, characterized in that the anionizing agent is an alkali metal alkoholate in the corresponding alcohol.

7. Process according to claim 6, characterized in that the operation is performed at a temperature below 0°C.

8. Process according to claim 6, characterized in

that the operation is performed at a temperature below −30°C.

9. Process according to claim 1, characterized in that the anionizing agent and the halogenating agent are added to the product of general formula (IIa) or (IIb) in solution in an organic solvent.

10. Process according to claim 1, characterized in that the anionization of the product of general formula (IIa) or (IIb) is performed first and the halogenating agent is then added.

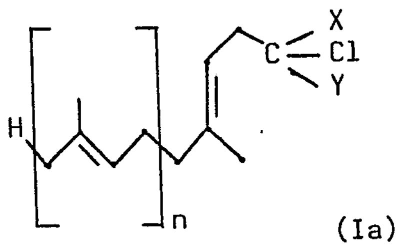

(Ia)

ou

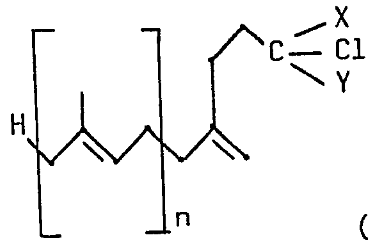

(Ib)

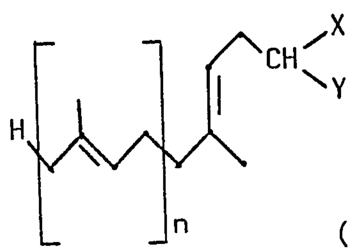

(IIa)

ou

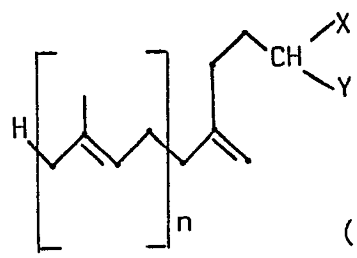

(IIb)

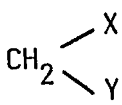

(III)

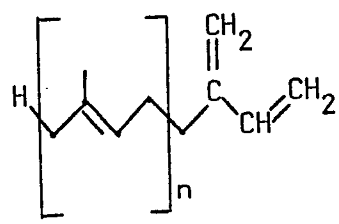

(IV)

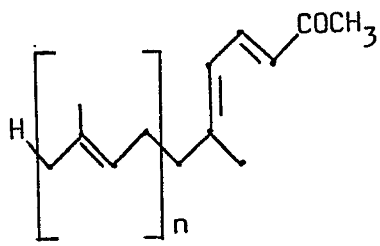

(V)

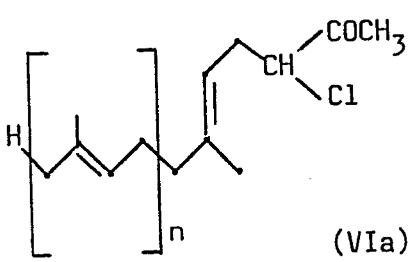

(VIa)

ou

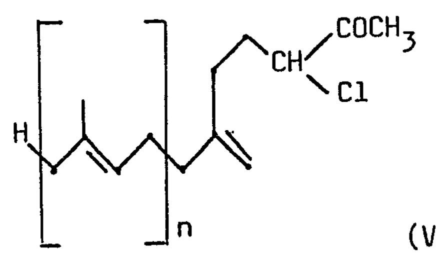

(VIb)